(19) Europäisches Patentamt
European Patent Office
Office européen des brevets

(11) Numéro de publication: **0 022 396 B1**

(12) **FASCICULE DE BREVET EUROPEEN**

(45) Date de publication du fascicule du brevet: **08.12.82**

(51) Int. Cl.³: **C 07 C 179/10**, C 07 C 179/12, C 07 C 178/00

(21) Numéro de dépôt: **80400937.1**

(22) Date de dépôt: **24.06.80**

(54) **Nouveau procédé de préparation d'acides percarboxyliques.**

(30) Priorité: **09.07.79 FR 7917720**

(43) Date de publication de la demande: **14.01.81 Bulletin 81/2**

(45) Mention de la délivrance du brevet: **08.12.82 Bulletin 82/49**

(84) Etats contractants désignés: **BE CH DE FR GB IT LI LU NL SE**

(56) Documents cités:
**BE-A-559 665**
**FR-A-2 038 575**
**FR-A-2 309 552**

(73) Titulaire: **P C U K PRODUITS CHIMIQUES UGINE KUHLMANN**, Service Propriété Industrielle Tour Manhattan, F-92087 Paris La Defense 2 Cédex 21 (FR)

(72) Inventeur: **Schirmann, Jean-Pierre, 49 Chemin de la Glaciére, F-69600 Oullins (FR)**

**EP 0 022 396 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Nouveau procédé de préparation d'acides percarboxyliques

La présente invention concerne un nouveau procédé simple de préparation d'acides percarboxyliques particulièrement intéressant pour l'oxydation sélective de composés organiques.

Il est connu depuis les travaux de Ans et al. (Ber. 45, 1845, 1912) que le peroxyde d'hydrogène réagit avec les acides carboxyliques aliphatiques pour former des acides percarboxyliques selon une réaction équilibrée qui peut être formulée ainsi:

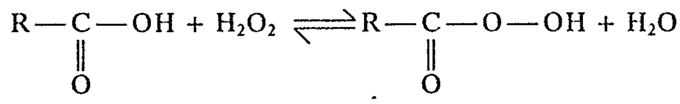 (I)

Comte-tenu de l'instabilité des peroxyacides, cette réaction est en général réalisée à basse température. Dans ces conditions l'état d'équilibre n'est atteint qu'après plusieurs heures de réaction et une telle durée est prohibitive pour un procédé industriel. Il est donc nécessaire d'avoir recours à un catalyseur. Un seul type de catalyseur a été proposé jusqu'à maintenant: les acides minéraux forts tels que l'acide sulfurique, l'acide méthanesulfonique, les acides arylsulfoniques, l'acide phosphorique, les esters phosphoriques acides, l'acide trifluoroacétique ainsi que les résines cationiques acides telles que »Dowex 50« ou »Amberlite IR-120«.

Ce processus catalytique a donné lieu à de nombreuses études (D. Swern, »Organic Peroxides«, Wiley Interscience, 1970, Vol. 1, pages 313–369 et pages 428–439) dont il ressort clairement que la première étape de la réaction correspond à la protonation de la fonction acide, entraînant la formation d'une structure oxonium capable de réagir avec $H_2O_2$ pour conduire après déshydratation à l'acide percarboxylique selon le schéma:

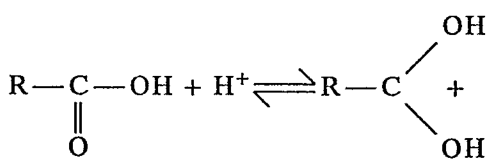 (II)

$$H_2O_2 \rightleftharpoons HOO^- + H^+ \qquad (III)$$

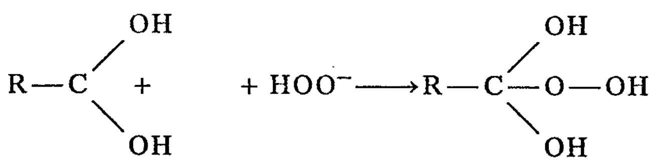 (IV)

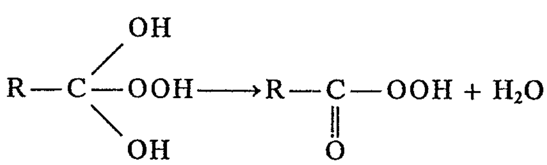 (V)

Le peroxyde d'hydrogène est le plus souvent utilisé sous forme de solutions aqueuses commerciales contenant de 30 à 70% d'eau. Par ailleurs la réaction fournit aussi de l'eau. Il en résulte que l'état d'équilibre est atteint bien avant que le peroxyde d'hydrogène soit totalement transformé. Dans ces conditions le produit de la réaction est en fait un mélange d'acide, de peroxyde d'hydrogène, de peracide, d'eau et d'acide fort. L'utilisation d'un tel mélange comme moyen d'oxydation en chimie organique conduit de ce fait à des rendements très moyens.

Pour pallier cet inconvénient, il a été proposé d'opérer en présence d'un très large excès d'acide carboxylique de façon à déplacer l'équilibre vers la droite. C'est ainsi qu'en utilisant 10 moles d'acide acétique pour une mole de peroxyde d'hydrogène, on peut atteindre un taux de conversion de 90% du peroxyde d'hydrogène en acide peracétique. L'utilisation d'un tel excès ne permet d'obtenir que des solutions très diluées de peracide et entraîne souvent des pertes de rendements du fait de réactions parasites, sans compter les problèmes de séparation ultérieure des produits de la réaction.

Aussi a-t-on proposé comme dans les brevets des Etats-Unis n° 2.877.266 et n° 2.814.641 et dans le brevet belge n° 559 665, de n'opérer qu'avec un très léger excès d'acide carboxylique, mais d'opérer en présence d'un catalyseur acide minéral fort et d'un entraineur azéotropique pour éliminer l'eau et ainsi déplacer l'équilibre (I) vers la droite. Une telle pratique est effectivement excellente quant au rendement en acide percarboxylique par rapport au peroxyde d'hydrogène engagé. Comparée aux techniques précédentes, on pourrait espérer que cette technique donne dans les réactions d'oxydation en chimie organique de bons rendements. Cela n'est guère le cas et le rendement peut

être d'autant plus médiocre que l'acide fort catalyseur engendre bien souvent des réactions parasites. Il est bien connu par exemple que dans les réactions d'époxydation des oléfines par les peracides, l'époxyde formé est facilement ouvert et transformé en mono ou diester sous l'effet des catalyseurs acides forts.

Il est vrai que l'acide fort peut être avantageusement neutralisé, mais alors le sel correspondant est en général insoluble dans le milieu et pose des problèmes de séparation non négligeables sur le plan pratique. Parfois même il est aussi bon catalyseur de réactions parasites que l'acide lui-même.

C'est pourquoi on a proposé, récemment, comme dans les brevets français n° 2.359.132 et n° 2.300.085, une méthode de préparation de solutions organiques d'acides percarboxyliques en deux étapes qui consiste à faire réagir une solution aqueuse contenant de 10 à 45% d'acide sulfurique, de 20 à 35% de peroxyde d'hydrogène avec de l'acide propionique, puis à extraire l'acide perpropionique à l'aide d'un solvant tel que le benzène ou le dichloropropane. La phase aqueuse doit être concentrée pour éliminer l'eau apportée par $H_2O_2$ et par la réaction. La phase organique est lavée pour éliminer $H_2SO_4$, puis séchée par distillation azéotropique par exemple. Cette solution permet effectivement d'obtenir une solution organique d'acide perpropionique anhydre et débarrassée d'acide sulfurique. Cependant, il s'agit d'une technique lourde à mettre en oeuvre et par conséquent coûteuse.

D'autre part le brevet français n° 2.038.575 décrit un procédé d'oxydation indirecte et cyclique de composés organiques accepteurs d'oxygène ou donneurs d'électrons par l'intermédiaire d'un vecteur d'oxygène, obtenu par oxydation d'un support minéral, qui peut être un métalloïde comme le sélénium, à l'aide d'un composé peroxygéné, tel que le peroxyde d'hydrogène. L'oxydation est conduite de préférence dans le xylène, avec entrainement azéotropique continu de l'eau de réaction et de l'eau apportée par les réactifs. Ce procédé, qui s'applique particulièrement à l'oxydation de la méthyl-2-pyridine, du paraldéhyde, de l'acétone, de l'acétophénone, de l'hydroquinone et du monosulfure de tétraéthylthiurame, implique trois étapes successives:

— l'oxydation par le peroxyde d'hydrogène du métalloïde en oxyde de métalloïde,
— l'oxydation du composé organique par l'oxyde métalloïdique, qui est réduit à l'état de métalloïde qui précipite,
— le recyclage du métalloïde précipité à la première étape.

Il est donc lui aussi compliqué à mettre en oeuvre et par conséquent coûteux.

Poursuivant ses travaux dans le domaine de l'utilisation du peroxyde d'hydrogène en chimie organique, la demanderesse vient de découvrir qu'il est possible d'obtenir une solution organique anhydre d'acide percarboxylique vierge de toute trace d'acide minéral fort, en faisant réagir l'acide carboxylique et le peroxyde d'hydrogène, en présence de nouveaux catalyseurs constitués par un oxyde métalloïdique et d'un entraineur azéotropique de façon à éliminer continuellement du milieu réactionnel l'eau apportée par la solution aqueuse de peroxyde d'hydrogène ainsi que l'eau résultant de la réaction.

Les oxydes métalloïdiques qui entrent dans le cadre de la présente invention sont ceux du sélénium, du tellure, de l'arsenic, de l'antimoine, du bismuth et du bore. A titre d'exemples non limitatifs, on peut citer les oxydes suivants: $SeO_2$, $TeO_2$, $As_2O_3$, $As_2O_5$, $Sb_2O_5$, $Bi_2O_3$, $SeO_2$, $B_2O_3$.

Les acides carboxyliques concernés par l'invention sont les acides carboxyliques aliphatiques solubles dans l'eau, c'est-à-dire les acides formique, acétique, propionique, butyriques.

L'entraineur azéotropique peut être choisi de façon avantageuse parmi les solvants de point d'ébullition inférieur à 100° C et formant un hétéroazéotrope avec l'eau. On peut citer à titre d'exemples non limitatifs des solvants chlorés tels que le chloroforme, le tétrachlorure de carbone, le chlorure de méthylène, le dichloro-1,2-éthane, le dichloropropane, des solvants hydrocarbonés tels que le cyclohexane, le benzène, le toluène, des esters tels que les formiates, acétates, propionates, butyrates, isobutyrates de méthyle, d'éthyle, de propyle, d'isopropyle, de n-butyle.

Le peroxyde d'hydrogène peut être mis en oeuvre dans le cadre de l'invention aussi bien sous forme anhydre, que sous forme de solution aqueuse commerciale titrant de 30 à 70% en poids.

Le procédé conforme à l'invention consiste donc à mettre en contact l'acide carboxylique, l'entraineur azéotropique, le catalyseur et le peroxyde d'hydrogène en éliminant continuellement l'eau du milieu réactionnel par distillation azéotropique.

La température à laquelle est réalisée la réaction est comprise entre 40°C et 100°C et préférentiellement entre 40° C et 70° C. Selon la température choisie et le système réactionnel mis en oeuvre, l'élimination pourra se faire en opérant à pression atmosphérique ou sous pression réduite. La pression peut donc varier entre 20 mm de mercure et 760 mm de mercure.

La durée de la réaction dépend de la nature du catalyseur, de la nature de l'acide carboxylique, de la nature de l'entraineur azéotropique et de la température choisie. Elle peut aller de quelques minutes à plusieurs heures. Les réactifs peuvent être engagés en quantités équimoléculaires, mais on peut aussi utiliser un excès ou un défaut molaire de l'un ou l'autre des réactifs. A titre indicatif, on peut engager de 0,1 à 10 moles d'acide carboxylique par mole de peroxyde d'hydrogène, mais on engage préférentiellement de 1 à 5 moles.

Le catalyseur est utilisé à raison de 0,001 à 0,1 mole d'oxyde métalloïdique par mole de peroxyde

d'hydrogène. On préfère cependant un rapport molaire compris entre 0,001 et 0,01 mole par mole de peroxyde d'hydrogène engagé.

La quantité de solvant entraineur azéotropique est comprise entre 50 et 75% en poids du mélange réactionnel, de telle sorte que l'on puisse régler à souhait le point d'ébullition du mélange et éliminer de façon efficace l'eau.

Les réactifs peuvent être utilisés sous leur forme commerciale habituelle. Le peroxyde d'hydrogène en particulier peut être mis en oeuvre sous forme de solutions aqueuses commerciales titrant de 30 à 70% en poids. Il peut être avantageux d'ajouter au mélange réactionnel un produit stabilisant du peroxyde d'hydrogène, tels que polyphosphates, dérivés de l'acide éthylènediamine-tétraacétique (EDTA), etc...

La solution d'acide percarboxylique ainsi obtenue peut alors servir à réaliser l'oxydation de très nombreux composés organiques tels que oléfines, cétones, amines, composés aromatiques, dérivés soufrés, etc..., au cours d'une deuxième opération. Cependant il n'est pas toujours nécessaire d'avoir recours à cette procédure et l'on peut parfois avantageusement réaliser les deux opérations en même temps, c'est-à-dire la synthèse du peracide et sa consommation immédiate par la molécule à oxyder. Cela constitue une variante du procédé conforme à l'invention. C'est ainsi que lorsque le composé organique que l'on veut oxyder par l'acide percarboxylique, forme un hétéroazéotrope avec l'eau, il peut être utilisé comme entraineur azéotropique et dans le même temps réagir avec l'acide percarboxylique au fur et à mesure de la formation de celui-ci. On peut citer à titre d'exemple l'époxydation du cyclohexène ou du chlorure d'allyle par l'acide peracétique ou l'acide perpropionique. Une telle procédure est particulièrement simple à mettre en oeuvre et est particulièrement sécuritive puisqu'elle permet d'éviter toute accumulation de peracide dans le milieu réactionnel.

Dans le cadre de cette variante, si le composé à oxyder ne forme pas d'hétéroazéotrope avec l'eau, il est bien sûr tout-à-fait possible d'opérer en présence d'un solvant entraineur azéotropique.

Les exemples suivants illustrent de façon non limitative la présente invention.

## Exemples 1 à 9

Dans un réacteur de 250 $cm^3$ équipé d'une colonne de distillation de 5 plateaux Oldershaw surmontée d'un condenseur à reflux, on place 50 g d'acide propionique, 70 g de solvant entraineur azéotropique, 0,2 g de catalyseur. Ce mélange est porté à reflux, puis on introduit progressivement 0,1 mole de peroxyde d'hydrogène sous forme de solution aqueuse à 70% en poids. Le condenseur est conçu de telle façon que seule la phase organique condensée est refluée dans la colonne, la phase aqueuse décantée étant soutirée de façon continue. Les conditions de réaction et les résultats sont consignés dans le tableau suivant.

## Exemple 10

Dans un réacteur de 500 $cm^3$ équipé d'une colonne de distillation de 10 plateaux Oldershaw surmontée d'un condenseur à reflux du même type que celui décrit ci-dessus, on place 125 g d'acide propionique, 175 g de dichloro-1,2 éthane, 0,5 g d'oxyde de bore $B_2O_3$ et 0,1 g phosphate disodique. On porte à reflux sous une pression de 150 mm de Hg. La température du milieu réactionnel est de 50°C. On additionne progressivement 0,3 mole de peroxyde d'hydrogène sous forme de solution aqueuse à 70% en poids. Après deux heures de réaction au cours desquelles l'eau est éliminée de façon continuelle par distillation azéotropique, on dose dans le milieu 0,24 mole d'acide perpropionique ainsi que 0,027 mole de peroxyde d'hydrogène, alors que la phase aqueuse distillée contient 0,032 mole de peroxyde d'hydrogène.

## Exemple 11

On répète la même expérience que dans l'exemple 7 en remplaçant l'acide propionique par l'acide acétique. Après 60 minutes de réaction, on dose dans le milieu réactionnel 0,07 mole d'acide peracétique et 0,026 mole de peroxyde d'hydrogène alors que 0,004 mole sont passées dans la phase aqueuse du distillat.

## Exemple 12

A 120 g de solution d'acide perpropionique préparée selon l'exemple 10 et contenant 0,09 mole de peracide, on ajoute à température ordinaire 8,2 g de cyclohexène. Après une heure de réaction, on dose par chromatographie en phase gazeuse 0,08 mole d'époxyde du cyclohexène.

| Exemple | Catalyseur | Solvant | T °C | Pression mm Hg | Durée mn | $H_2O_2$ restante m · mole | Peracide formé m · mole | $H_2O_2$ distillée |
|---|---|---|---|---|---|---|---|---|
| 1 | – | Cyclohexane | 93° | 760 | 30 | 14,1 | 13 | 34,3 |
| 2 | $As_2O_5$ | Benzène | 94° | 760 | 60 | 2 | 20 | 20 |
| 3 | $As_2O_5$ | Dichloréthane | 94° | 760 | 60 | 19 | 58 | 11 |
| 4 | $B_2O_3$ | Cyclohexane | 88° | 760 | 30 | 3 | 49,5 | 20,5 |
| 5 | $B_2O_3$ | Dichloréthane | 94° | 760 | 30 | 6 | 74 | 7 |
| 6 | $B_2O_3$ | Dichloréthane | 70° | 350 | 60 | 4 | 80 | 4 |
| 7 | $B_2O_3$ | Dichloréthane | 50° | 150 | 60 | 27 | 62,5 | 15 |
| 8 | $SeO_2$ | Dichloréthane | 94° | 760 | 30 | 3 | 36 | 6 |
| 9 | $Sb_2O_5$ | Dichloréthane | 95° | 760 | 60 | 20 | 65 | 22 |

Exemple 13

Dans un réacteur tel que décrit à l'exemple 1, on place 50 g d'acide propionique, 50 g de chlorure d'allyle ainsi que 0,1 g d'oxyde d'arsenic $As_2O_5$. On porte à reflux et la température du milieu réactionnel se fixe à 64° C. On ajoute en 15 minutes, 0,053 mole de peroxyde d'hydrogène sous forme de solution aqueuse à 70% et l'on élimine en continu l'eau apportée par $H_2O_2$ et celle formée au course de la réaction. Après une heure de réaction, on dose dans le milieu réactionnel 0,034 mole d'épichlorhydrine, 0,008 mole d'acide perpropionique et 0,006 mole de peroxyde d'hydrogène alors que le distillat contient 0,004 mole de peroxyde d'hydrogène.

Exemple 14

Dans un réacteur tubulaire de 15 m de longueur et de diamètre ∅: 2 mm, on introduit en continu simultanément après passage dans un mélangeur 100 g/h de solution d'acide perpropionique préparé selon l'exemple 10 et titrant 6,6% de peracide et 0,25% de peroxyde d'hydrogène, ainsi que 21 g/h de propylène. La température du réacteur est maintenue à 50° C. La pression dans le réacteur est de 8 bars. A la sortie du réacteur, on procède en continu à la décompression du mélange réactionnel. La phase gazeuse est lavée à l'eau dans une colonne de lavage pour récupérer l'oxyde de propylène entrainé. La phase liquide est refroidie. L'analyse des produits de la réaction montre qu'il sort du réacteur, 0,001 mole par heure d'acide perpropionique et qu'il s'est formé 4 g/h d'oxyde de propylène.

**Revendications**

1. Procédé de préparation d'acide percarboxylique par action du peroxyde d'hydrogène sur un acide carboxylique aliphatique miscible à l'eau, en phase liquide homogène, à une température comprise entre 40 et 100° C, en présence d'un catalyseur et d'un solvant, représentant de 50 à 75% en poids du mélange réactionnel et permettant d'éliminer de façon continue par distillation azéotropique l'eau introduite avec le peroxyde d'hydrogène ainsi que l'eau formée au cours de la réaction, caractérisé en ce que l'on utilise comme catalyseur un oxyde métalloïdique à raison de 0,001 à 0,1 mole par mole de peroxyde d'hydrogène.

2. Procédé selon la revendication 1, caractérisé en ce que le catalyseur est l'oxyde de bore.

3. Procédé selon la revendication 1, caractérisé en ce que le catalyseur est un oxyde d'arsenic.

4. Procédé selon la revendication 1, caractérisé en ce que le catalyseur est l'oxyde de sélénium.

5. Procédé selon l'une quelconque des revendications 1 à 4, caractérisé en ce que l'entraîneur azéotropique est un solvant chloré.

6. Procédé selon l'une quelconque des revendications 1 à 4, caractérisé en ce que l'entraîneur azéotropique est le benzène.

7. Procédé selon l'une quelconque des revendications 1 à 6, caractérisé en ce que l'acide carboxylique aliphatique miscible à l'eau est l'acide acétique.

8. Procédé selon l'une quelconque des revendications 1 à 6, caractérisé en ce que l'acide carboxylique aliphatique miscible à l'eau est l'acide propionique.

9. Procédé selon l'une quelconque des revendications 1 à 8, caractérisé en ce que l'entraîneur azéotropique est un composé organique susceptible de réagir avec l'acide percarboxylique au fur et à mesure de sa formation dans le milieu réactionnel.

10. Procédé selon la revendication 9, caractérisé en ce que l'entraîneur azéotropique est une oléfine.

11. Procédé selon la revendication 10, caractérisé en ce que l'entraîneur azéotropique est le cyclohexène.

12. Procédé selon la revendication 10, caractérisé en ce que l'entraîneur azéotropique est le chlorure d'allyle.

**Patentansprüche**

1. Verfahren zur Herstellung von Percarbonsäure durch Einwirkung von Wasserstoffperoxid auf eine aliphatische, mit Wasser mischbare Carbonsäure in homogener Phase bei einer Temperatur zwischen 40 und 100° C in Gegenwart eines Katalysators und eines Lösungsmittels, das 50 bis 75 Gew.-% des Reaktionsgemisches ausmacht und durch azeotrope Destillation die kontinuierliche Entfernung des mit dem Wasserstoffperoxid eingebrachten und bei der Reaktion gebildeten Wassers ermöglicht, dadurch gekennzeichnet, daß man als Katalysator ein Halbmetalloxid in einer Menge von 0,001 bis 0,1 Mol pro Mol Wasserstoffperoxid verwendet.

2. Verfahren nach Anspruch 1, dadurch gekennzeichnet, daß der Katalysator Boroxid ist.

3. Verfahren nach Anspruch 1, dadurch gekennzeichnet, daß der Katalysator ein Arsenoxid ist.

4. Verfahren nach Anspruch 1, dadurch gekennzeichnet, daß der Katalysator Selenoxid ist.

5. Verfahren nach einem der Ansprüche 1 bis 4, dadurch gekennzeichnet, daß das azeotrope Schleppmittel ein chloriertes Lösungsmittel ist.

6. Verfahren nach einem der Ansprüche 1 bis 4, dadurch gekennzeichnet, daß das azeotrope Schleppmittel Benzol ist.

7. Verfahren nach einem der Ansprüche 1 bis 6, dadurch gekennzeichnet, daß die mit Wasser mischbare, aliphatische Carbonsäure Essigsäure ist.

8. Verfahren nach einem der Ansprüche 1 bis 6, dadurch gekennzeichnet, daß die mit Wasser mischbare aliphatische Carbonsäure Propionsäure ist.

9. Verfahren nach einem der Ansprüche 1 bis 8, dadurch gekennzeichnet, daß das azeotrope Schleppmittel eine organische Verbindung ist, die mit der Percarbonsäure im Maße ihrer Bildung im Reaktionsgemisch reagieren kann.

10. Verfahren nach Anspruch 9, dadurch gekennzeichnet, daß das azeotrope Schleppmittel ein Olefin ist.

11. Verfahren nach Anspruch 10, dadurch gekennzeichnet, daß das azeotrope Schleppmittel Cyclohexen ist.

12. Verfahren nach Anspruch 10, dadurch gekennzeichnet, daß das azeotrope Schleppmittel Allylchlorid ist.

**Claims**

1. Process for the preparation of a percarboxylic acid by reacting hydrogen peroxide with a water-miscible aliphatic carboxylic acid, in a homogeneous liquid phase, at a temperature of between 40 and 100°C, in the presence of a catalyst and of a solvent representing from 50 to 75% by weight of the reaction mixture and making it possible continuously to remove, by azeotropic distillation, the water introduced with the hydrogen peroxide and also the water formed during the reaction, characterised in that the catalyst used is a metalloid oxide in an amount of 0,001 to 0.1 mol per mol of hydrogen peroxide.

2. Process according to Claim 1, characterised in that the catalyst is boron oxide.

3. Process according to Claim 1, characterised in that the catalyst is an arsenic oxide.

4. Process according to Claim 1, characterised in that the catalyst is selenium oxide.

5. Process according to any one of Claims 1 to 4, characterised in that the azeotropic distillation agent is a chlorinated solvent.

6. Process according to any one of Claims 1 to 4, characterised in that the azeotropic distillation agent is benzene.

7. Process according to any one of Claims 1 to 6, characterised in that the water-miscible aliphatic carboxylic acid is acetic acid.

8. Process according to any one of Claims 1 to 6, characterised in that the water-miscible aliphatic carboxylic acid is propionic acid.

9. Process according to any one of Claims 1 to 8, characterised in that the azeotropic distillation agent is an organic compound capable of reacting with the percarboxylic acid as it is formed in the reaction medium.

10. Process according to Claim 9, characterised in that the azeotropic distillation agent is an olefine.

11. Process according to Claim 10, characterised in that the azeotropic distillation agent is cyclohexene.

12. Process according to Claim 10, characterised in that the azeotropic distillation agent is allyl chloride.